# EUROPEAN PATENT SPECIFICATION

(11) **EP 0 580 706 B1**
(45) Date of publication and mention of the grant of the patent: **28.02.1996**
(21) Application number: 92909203.9
(22) Date of filing: 13.04.1992
(51) Int. Cl.: A61M 5/24

(54) **DEVICE FOR THE ADMINISTRATION OF DRUGS**
VORRICHTUNG ZUR ABGABE VON MEDIKAMENTEN
DISPOSITIF POUR L'ADMINISTRATION DE MEDICAMENTS

(30) Priority: 18.04.1991 IT TO910091 U
(43) Date of publication of application: 02.02.1994
(73) Proprietor: BRACCO S.p.A., I-20134 Milano (IT)
(72) Inventor: TOMELLINI, Giorgio, I-16146 Genova (IT); ROLLANDI, Gian, Andrea, I-16125 Genova (IT)
(74) Representative: Bosotti, Luciano
(86) International application number: EP9200826
(87) International publication number: WO9218178

(56) References cited:
- EP-A- 0 206 971
- DE-U- 9 003 505
- FR-A- 1 551 012
- FR-A- 2 262 535
- US-A- 3 994 296

## Description

The present invention relates generally to devices for the administration of drugs and relates in particular to an improvement which can be made in the solutions described in the prior Italian utility model applications 15156-B/89 and TO91UO00078, to which DE-U-9 003 505 and WO 92/18177 correspond, respectively.

Specifically, the present invention relates to a device having the features according to the preamble of claim 1, which is known, e.g. from EP-A-0 206 971. A substantially similar arrangement is known from FR-A-1 551 012.

The underlying problem of the present invention is to improve the prior art solutions especially as regards the possibility of retracting the container acting as a plunger during administration of goods, achieving a good fluid-tight sealing of the connection between the needle and the container, and avoiding any contact of the needle tip with the user.

The invention, having the characteristics claimed specifically in the characterising portion of Claim 1, will now be described, purely by way of non-limiting example, with reference to the appended drawings (corresponding substantially to the Figures 1-4 appended to the specification of Italian application 15156-B/89 or DE-U-9 003 505) in which:
Figures 1 and 2 are axial sections of the two elements (housing and container) which together define a device for the administration of drugs according to the invention,
Figure 3 illustrates, in greater detail, the salient element of the invention, that is the structure of the unit for coupling the said housing and container, and
Figure 4 shows these elements coupled in their position of use.

The housing 10, usually made from a plastics material suitable for medical or pharmaceutical uses, has a generally tubular structure which can be seen to comprise an outer jacket 12 and an inner jacket 13. These jackets 12, 13 together define an annular chamber 14 closed at one end by a so-called end wall 11 provided with an attachment formation 38 for the connection, for example, of a hypodermic needle, not illustrated.

Within the inner jacket 13, in a central axial position relative to the housing 10, is a needle 20 which, in the embodiment illustrated, extends through practically the entire length of the housing 10 and terminates at its end opposite the end wall 11 ("open" end of the housing 10) in a sharp tip 21. The length of the needle 20 is selected so that the sharp end 21 does not project from the housing 10 and is thus substantially aligned with (or slightly inset from) the open end of the housing 10 where the latter has an external flange 16 which can serve as a hand grid for use of the device.

In correspondence with the end wall 11, the needle 20 is supported by a frusto-conical or annular diaphragm or partition which defines a central outlet aperture towards the hypodermic needle. At the opposite end of the housing 10, the needle 20 is supported by a further partition 100 which closes the inner jacket 13 in correspondence with the open end of the housing 10 and is provided with an attachment formation 102. Both the partition 100 and the formation 102 are preferably made from the same material as that forming the housing 10 overall. The attachment formation 102 is generally mushroom-shaped, with a generally T-shape in transverse section.

Whenever, however, it is wished to make use of the solution described in the prior Italian application T091UO00078 or WO 92/18177, the needle may instead be made so as not to extend as far as the end wall 11. In this case (not illustrated here) the needle 20 stops in correspondence with a partition which defines a terminal chamber within the inner jacket 13 intended to contain one of the components (usually a powder) of a two-component drug.

The other element of the device, that is the container 30, is defined by a generally cylindrical cup-shaped body of plastics material suitable for pharmaceutical use. This cup-shaped body is closed at its open end by a body 31 intended to act both as a stopper and as a piston which can slide in a substantially sealed manner within the container 30. This latter is intended to receive the drug to be supplied or (in the case of the solution described in Italian application TO91UO00078 or WO 92/18177) the liquid component of a two-component drug to be supplied. When the container is closed, the piston 31 is located close to the open end of the container itself.

The piston 31 has an axial through-hole 33 closed by a diaphragm 34 intended to be pierced by the sharp end 21 of the needle 20. In general, the piston 31 is of soft material such as rubber and is held in a fixed position until the moment of use by known means, for example a small disc (not illustrated) which acts as a seal and as a stop.

The salient characteristic of the solution of the invention is the fact that the piston 31 (immediately above the diaphragm 34, in the non-limiting embodiment illustrated here) has a recess 104 of complementary shape to that of the formation 102 provided in the housing 10. In the case of the recess 104 in the example illustrated here, therefore, this is a negative-mushroom shape having a lower, generally-cylindrical chamber and an upper, narrower neck portion which opens to the exterior of the container 30.

A cup-shaped cover 36 protects the open end of the container 30 until the device is used. Usually the latter is packaged in the form of a kit whose two component parts are the housing 10 and the container 30.

During use, the cup-shaped cover 36 is removed, together with any locking and sealing means associated therewith (not illustrated) which retain the piston 31 at the open end of the container 30.

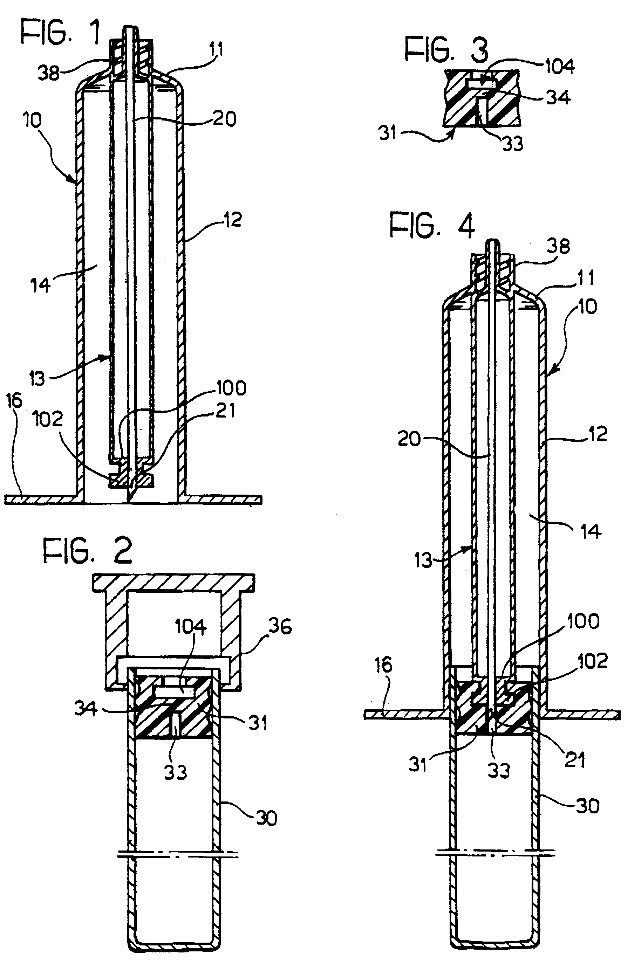

At this point, the container 30 is engaged with the open end of the housing 10 in the condition illustrated schematically in Figure 4, that is, with the peripheral wall of the container 30 penetrating the annular chamber 14 defined between the jackets 12 and 13 of the housing 10. This result may be achieved by fitting the piston 31 onto the end portion of the inner jacket 13 and then pressing to cause the formation 102 to enter the recess 104 to form a coupling between the piston 31 and the central part of the housing 10.

This result is generally achieved by the penetration and snap engagement of the formation 102 (male formation) in the recess 104 (female formation) which is allowed by the generally yielding nature of the soft material of which the piston 31 is made: this means that the lip which defines the narrow mouth of the recess 104 yields elastically as it is penetrated by the formation 102.

A coupled condition is thus achieved by snap engagement with precise complementary-form coupling as shown schematically in Figure 4.

As a result of the movement which causes this coupling, the needle 20 and more particularly the pointed end 21 thereof pierces the diaphragm 34 so as to bring the chamber within the container 30 into contact with the interior of the needle 20.

At this point, a hypodermic needle may be fitted on to the spigot 38 and, when the flanges 16 and the housing 10 have been grasped by two fingers, the pressure of the thumb on the end wall of the container 30 enables this to be pushed into the annular chamber 14 in the housing 10 with the consequent forcing of the piston 31 back into the container 30. The result of this is that the liquid in the container 30 is expelled through the needle and hence through the hypodermic needle fitted on the spigot 38.

In the solution of application TO91UO00078 or WO 92/18177, the liquid component of the drug flows from the container 30 into an end chamber containing the powdered component of the drug so as to cause the components to mix and is then expelled to the exterior of the device.

The firm attachment of the piston 31 to the central part of the housing 10 which is thus achieved in any case enables the container 30 to be retracted when this is necessary, for example during an injection. In addition, the generally soft character of the material constituting the stopper 31 also achieves good fluid-tight sealing of the connection between the container 30 and the needle 20.

The same structure may also be used for the supply of infusions as indicated in Italian application 15156-B/89 or DE-U-9 003 505, (Fig.6).

## Claims

1. A device for the administration of drugs comprising a first element (10) and a second element (30) intended to be coupled together in use, in which:
- the second element (30) is a container for fluid which is closed by a piston (31) which can be made pervious (34) to fluids and which is movable within the container (30) itself in order to cause the fluid to be expelled from the container (30),
- the first element (10) includes an inner jacket (13) fixedly supporting withdrawal means (20, 21) which can be coupled to the piston (31) in order to make it pervious (34) and to cause its movement into the container (30),
- the withdrawal means (20, 21) and the piston (31) have associated therewith complementary male (102) and female (104) attachment formations, respectively, which can form a coupling when the first element (10) and the second element (30) are coupled together in use,
- the male formation (102) is rigid while the female formation (104) is deformable, and
- the withdrawal means comprise a needle (20) with a tip (21); the tip (21) being surrounded by and protruding from the respective male complementary formation (102),
characterised in that:
- said complementary male (102) and female (104) formations have complementary shapes, whereby they can form a strong form coupling when the first (10) and the second (30) element are coupled together in use, thus enabling the second element (30) to be retracted in use, and,
- said tip (21) and said male formation (102) are located in a retracted position in the region of the first element (10) in order to avoid any contact of the tip (21) with the user.

2. A device according to claim 1, characterised in that the complementary formations (102, 104) have a generally mushroom-shape and/or a T-section structure;

3. A device according to claim 1 or claim 2, characterised in that the piston (31) has a thin portion (34) intended to be pierced by the tip in order to render the piston (31) pervious.

4. A device according to any one of the preceding claims, characterised in that the first element (10) and the second element (30) have generally cylindrical structures and in that the complementary formations (102, 104) are located generally axially of the said elements (10, 20).

## Patentansprüche

1. Vorrichtung zur Abgabe von Medikamenten umfassend ein erstes Element (10) und ein zweites Element (30), welche für den Gebrauch aneinander kuppelbar sind, wobei:
- das zweite Element (30) ein Behälter für Fluid ist, welcher durch einen Kolben (31) verschlossen ist, der für Fluids durchlässig (34) gemacht werden kann und im Behälter (30) selbst bewegbar ist, um das Fluid aus dem Behälter (30) auszustoßen,
- das erste Element (10) einen inneren Mantel (13) umfaßt, der unverschiebbare Entnahmemittel (20, 21) trägt, die mit dem Kolben (31) kuppelbar sind, um ihn durchlässig (34) zu machen und seine Bewegung in den Behälter (30) hinein zu ermöglichen,
- den Entnahmemitteln (20, 21) und dem Kolben (31) sich ergänzende männliche (102) bzw. weibliche (104) Befestigungselemente zugeordnet sind, die eine Kupplung bilden können, wenn das erste Element (10) und das zweite Element (13) für den Gebrauch zusammengekuppelt werden,
- das männliche Element (102) starr ist, während das weibliche Element (104) verformbar ist, und
- die Entnahmemittel eine Nadel (20) mit einer Spitze (21) umfassen, wobei die Spitze (21) vom männlichen Ergänzungselement (102) umgeben ist und über dieses vorsteht, dadurch gekennzeichnet, daß
- das männliche (102) und weibliche (104) Ergänzungselement einander ergänzende Formen haben und somit einen festen Formschluß bilden können, wenn das erste (10) und das zweite (30) Element für den Gebrauch aneinander gekuppelt werden, wodurch das Zurückziehen des zweiten Elementes (30) für den Gebrauch möglich ist, und
- daß die Spitze (21) und das männliche Element (102) im Bereich des ersten Elementes (10) in einer inneren Stellung liegen, um jede Berührung der Spitze (21) mit dem Benützer zu vermeiden.

2. Vorrichtung nach Anspruch 1, dadurch gekennzeichnet, daß die Ergänzungselemente (102, 104) im allgemeinen eine Pilzform und/oder eine Struktur mit T-förmigem Querschnitt aufweisen.

3. Vorrichtung nach Anspruch 1 oder Anspruch 2, dadurch gekennzeichnet, daß der Kolben (31) einen dünnen Abschnitt (34) aufweist, der von der Spitze durchstoßbar ist, um den Kolben (31) durchlässig zu machen.

4. Vorrichtung nach einem der vorhergehenden Ansprüche, dadurch gekennzeichnet, daß das erste Element (10) und das zweite Element (30) im allgemeinen eine zylindrische Struktur aufweisen und daß die Ergänzungselemente (102, 104) im allgemeinen axial zu den Elementen (10, 20) liegen.

## Revendications

1. Dispositif pour l'administration de médicaments comprenant un premier élément (10) et un second élément (30) destinés à être couplés ensemble en utilisation, dans lequel :
- le second élément (30) est un conteneur pour un fluide qui est fermé par un piston (31) qui peut être rendu perméable (34) à des fluides et qui est mobile à l'intérieur du conteneur (30) lui-même afin de provoquer l'expulsion du fluide depuis le conteneur (30);
- le premier élément (10) inclut une chemise interne (13) qui supporte de façon fixe un moyen de retrait (20, 21) qui peut être couplé au piston (31) afin de le rendre perméable (34) et de provoquer son déplacement à l'intérieur du container (30);
- le moyen de retrait (20, 21) et le piston (31) se voient associer respectivement des formations de liaison mâle (102) et femelle (104) complémentaires qui peuvent former un couplage lorsque le premier élément (10) et le second élément (30) sont couplés ensemble en utilisation ;
- la formation mâle (102) est rigide tandis que la formation femelle (104) est déformable ; et
- le moyen de retrait comprend une aiguille (20) munie d'une extrémité (21), l'extrémité (21) étant entourée par la formation complémentaire mâle respective (102) et faisant saillie depuis celle-ci,
caractérisé en ce que :
- lesdites formations complémentaires mâle (102) et femelle (104) présentent des formes complémentaires de telle sorte qu'elles puissent former un couplage de type fort lorsque le premier élément (10) et le second élément (30) sont couplés ensemble en utilisation pour ainsi permettre au second élément (30) d'être retiré en utilisation ; et
- ladite extrémité (21) et ladite formation mâle (102) sont positionnées selon une position rétractée dans la région du premier élément (10) afin d'éviter un quelconque contact de l'extrémité (21) avec l'utilisateur.

2. Dispositif selon la revendication 1, caractérisé en ce que les formations complémentaires (102, 104) présentent la forme générale d'un champignon et/ou la structure d'une section en T.

3. Dispositif selon la revendication 1 ou 2, caractérisé en ce que le piston (31) comporte une partie mince (34) destinée à être percée par l'extrémité afin de rendre le piston (31) perméable.

4. Dispositif selon l'une quelconque des revendications précédentes, caractérisé en ce que le premier élément (10) et le second élément (30) présentent des structures de forme générale cylindrique et en ce que les formations complémentaires (102, 104) sont localisées de façon générale axialement par rapport auxdits éléments (10, 20).
